# FASCICULE DE BREVET EUROPEEN

(11) **EP 1 101 531 B1**
(45) Date de publication et mention de la délivrance du brevet: **25.06.2014**
(21) Numéro de dépôt: 00403224.9
(22) Date de dépôt: 17.11.2000
(51) Int. Cl.: B01J 38/58, B01J 37/16, C10G 2/00, C07C 1/04

(54) **Procédé d'activation d'un catalyseur de synthèse "Fischer-Tropsch"**
Verfahren zum Aktivieren eines Fischer-Tropschsynthesekatalysators
Process for activating a Fischer-Tropsch-synthesis catalyst

(30) Priorité: 22.11.1999 FR 9914762
(43) Date de publication de la demande: 23.05.2001
(73) Titulaire: Institut Français du Pétrole, 92852 Rueil-Malmaison Cedex (FR); AGIP PETROLI S.p.A., 00142 Roma (IT); ENI S.p.A., 00144 Rome (IT)
(72) Inventeur: Ducreux, Olivier, 78380 Bougival (FR); Marion, Marie-Claire, 69390 Vernaison (FR); Roy-Auberger, Magalie, 92500 Rueil Malmaison (FR); Lynch, John, 92500 Rueil Malmaison (FR)

(56) Documents cités:
- WO-A-93/00993
- WO-A-97/17137

## Description

La présente invention concerne un procédé d'activation d'un catalyseur utilisé dans un procédé de synthèse d'hydrocarbures à partir d'un mélange comprenant CO-H₂-(CO2), c'est à dire un mélange comprenant du monoxyde de carbone et de l'hydrogène et comprenant éventuellement du dioxyde de carbone, appelé gaz de synthèse.

Une telle synthèse permet généralement d'obtenir un mélange d'hydrocarbures linéaires saturés, de préférence essentiellement constitué d'hydrocarbures C5+ (c'est-à-dire possédant au moins 5 atomes de carbone par molécule).

### ART ANTERIEU R :

Il est connu de l'homme du métier que le gaz de synthèse peut être converti en hydrocarbures en présence de catalyseurs contenant des métaux de transition, de préférence le cobalt ou le fer. Cette conversion est connue dans la littérature sous le nom de synthèse Fischer-Tropsch.

De nombreuses méthodes ont été utilisées dans le passé soit pour activer la catalyseur neuf soit pour le régénérer après utilisation. Dans la plupart des cas, ce traitement d'activation, appelé aussi réduction, est réalisé sur le catalyseur neuf oxydé, et consiste en un traitement en température du catalyseur en présence d'hydrogène pur ou en présence d'un gaz contenant de l'hydrogène.

Ainsi les brevets EP-A-0 168 894, EP-A-0 152 652 montrent la possibilité d'améliorer les performances des catalyseurs en utilisant différentes conditions de pression partielle en hydrogène et de débits ; conditions qui peuvent être variables durant toute l'étape de réduction.

Le brevet US 5 168 091 décrit la possibilité de réduire le catalyseur sous hydrogène en maintenant une pression partielle d'eau inférieure à 0,1 MPa.

Les brevets EP-A-0 533 227 et EP-A-0 533 228 décrivent une méthode de réduction sous hydrogène qui en ajustant les pressions d'une part, et les débits d'autre part, permet d'optimiser l'activité des catalyseurs.

Les brevets WO 97/17137 et US 5 389 690 décrivent les avantages d'une réduction en phase slurry, c'est-à-dire avec un catalyseur en suspension dans une phase liquide constituée d'hydrocarbures. Le catalyseur est réduit en une seule étape, à une pression partielle en hydrogène supérieure à 1,5 MPa.

Enfin, le brevet WO 93/00993 décrit les avantages d'une activation en une seule étape, en présence de un gaz contenant du monoxyde de carbone et moins de 30 % d'hydrogène, de préférence en présence de monoxyde de carbone seul. Le catalyseur avant activation se présente sous forme oxydée. Le catalyseur ainsi obtenu présente une activité et une sélectivité en C5+ améliorée.

### RESUME DE L'INVENTION :

La présente invention concerne un procédé d'activation d'un catalyseur permettant de réaliser la synthèse d'hydrocarbures à partir d'un mélange comprenant du monoxyde de carbone, de l'hydrogène et éventuellement du dioxyde de carbone (synthèse Fischer-Tropsch). Une telle synthèse permet généralement d'obtenir un mélange d'hydrocarbures linéaires saturés.

Le procédé d'activation selon l'invention comprend au moins deux étapes:
- au moins une étape d'activation en présence d'hydrogène, ou d'un mélange d'hydrogène et de gaz inerte, et
- au moins une étape d'activation en présence de monoxyde de carbone ou d'un mélange de monoxyde de carbone et de gaz inerte,
ainsi qu'éventuellement une troisième étape d'activation réalisée soit en présence d'hydrogène ou d'un mélange d'hydrogène et de gaz inerte, soit en présence de monoxyde de carbone ou d'un mélange de monoxyde de carbone et de gaz inerte.

### DESCRIPTION DETAILLEE DE L'INVENTION:

La présente invention concerne un procédé d'activation d'un catalyseur actif dans la synthèse d'hydrocarbures à partir d'un mélange comprenant du monoxyde de carbone et
de l'hydrogène, éventuellement du dioxyde de carbone. Cette synthèse est également appelée synthèse Fischer-Tropsch.

Le procédé d'activation selon l'invention comprend au moins deux étapes:
- au moins une étape d'activation en présence de hydrogène, ou d'un mélange d'hydrogène et de gaz inerte, et
- au moins une étape d'activation en présence de monoxyde de carbone ou d'un mélange de monoxyde de carbone et de gaz inerte.

L'ordre de ces étapes est indifférent, mais durant l'activation au moyen du procédé selon l'invention, le catalyseur n'est pas mis en contact simultanément avec l'hydrogène et le monoxyde de carbone.

Un des modes de réalisation préféré du procédé d'activation selon l'invention est décrit ci-après.

Selon ce premier mode préféré, la première étape (étape 1) est réalisée en présence de hydrogène ou en présence d'un mélange d'hydrogène et de gaz inerte, à une température comprise entre 10°C et 700°C, de manière préférée entre 100°C et 600°C, et de manière plus préférée entre 200°C et 500°C, à une pression comprise entre 0,05 MPa et 30 MPa, de préférence entre 0,1 et 10 MPa, de manière plus préférée entre 0,1 et 2 MPa, à une vitesse volumétrique horaire comprise entre 20 et 100 000 h⁻¹ (volume de mélange par volume de catalyseur et par heure), de préférence entre 100 et 40 000 h⁻¹. La durée de la première étape est généralement supérieure à 10 minutes, de préférence comprise entre 1 et 24 heures, selon les conditions de débit et de température choisies.

Selon ce premier mode préféré, la deuxième étape (étape 2) est réalisée en présence de monoxyde de carbone ou en présence d'un mélange de monoxyde de carbone et de gaz inerte, à une température comprise entre 10°C et 700°C, de manière préférée entre 100°C et 600°C, et de manière plus préférée entre 180°C et 400°C, à une pression comprise entre 0,05 MPa et 30 MPa, de préférence entre 0,1 et 10 MPa, de manière plus préférée entre 0,1 et 2 MPa, à une vitesse volumétrique horaire comprise entre 20 et 100 000 h⁻¹ (volume de mélange par volume de catalyseur et par heure), de préférence entre 50 et 40 000 h⁻¹. La durée de la première étape est généralement supérieure à 10 minutes, de préférence comprise entre 1 et 24 heures, selon les conditions de débit et de température

Un autre mode de réalisation préféré du procédé d'activation selon l'invention consiste à réaliser une première étape en présence de monoxyde de carbone ou d'un mélange de monoxyde de carbone et de gaz inerte, et une deuxième étape en présence d'hydrogène ou d'un mélange d'hydrogène et de gaz inerte.

Les deux étapes de ce deuxième mode préféré sont réalisées dans les conditions décrites précédemment. La première étape de ce deuxième mode préféré est donc effectuée en présence de monoxyde de carbone ou d'un mélange de monoxyde de carbone et de gaz inerte, dans les mêmes conditions que l'étape 2 du premier mode préféré. De même, la deuxième étape en présence d'hydrogène ou d'un mélange d'hydrogène et de gaz inerte de ce deuxième mode préféré est effectuée dans les mêmes conditions que l'étape 1 du premier mode préféré.

Il est éventuellement possible d'ajouter une troisième étape au procédé d'activation selon l'invention. Cette troisième étape (étape 3) est également réalisée soit en présence d'hydrogène ou d'un mélange d'hydrogène et de gaz inerte, soit en présence de monoxyde de carbone ou d'un mélange de monoxyde de carbone et de gaz inerte, dans des conditions de température, de pression et de débit identiques à celles indiquées précédemment pour les étapes 1 ou 2.

Il est généralement préféré de réaliser à l'issue de chaque étape une purge sous gaz inerte, de manière à éliminer les traces d'hydrogène ou de monoxyde de carbone résiduel.

La totalité ou une partie de ces étapes peuvent être réalisées en phase gazeuse ou en phase liquide. Dans ce dernier cas, le catalyseur est mis en suspension dans un solvant inerte, par exemple une coupe paraffinique comprenant préférentiellement au moins un hydrocarbure ayant au moins 5, de manière plus préférée au moins 10 atomes de carbone par molécule, notamment et préférentiellement dans le cas où une réaction de synthèse d'hydrocarbures est mise en oeuvre juste après les étapes d'activation, et lorsque cette réaction est effectuée en présence d'une phase liquide comprenant de préférence au moins un hydrocarbure ayant au moins 5, de manière plus préférée au moins 10 atomes de carbone par molécule.

La conversion du gaz de synthèse en hydrocarbures intervient généralement après le procédé d'activation et est généralement opérée sous une pression totale habituellement comprise entre 0,1 et 15 MPa et de préférence entre 1 et 10 MPa, la température étant généralement comprise entre 150 et 350°C et de préférence entre 170 et 300°C.

La vitesse volumétrique horaire est habituellement comprise entre 50 et 50 000 h⁻¹, de préférence entre 100 et 20 000 h⁻¹, et de manière plus préférée entre 100 et 50 000 h⁻¹, et le rapport molaire H2:CO dans le gaz de synthèse est habituellement compris entre 1:2 et 5:1, de préférence entre 1,2:1 et 2,5:1.

Le procédé d'activation selon l'invention décrit précédemment peut être utilisé pour activer un catalyseur neuf à l'état oxydé, ou pour régénérer un catalyseur usagé à l'état oxydé ou au moins en partie à l'état réduit .

Le catalyseur est généralement utilisé soit sous la forme d'une poudre fine calibrée présentant une granulométrie généralement comprise entre 10 et 700 microns, soit sous la forme de particules de diamètre équivalent généralement compris entre 2 et 10 mm. Il est de préférence utilisé sous la forme d'une poudre fine calibrée lorsque le procédé d'activation selon l'invention est mis en oeuvre en phase liquide, et de préférence sous la forme de particules lorsque le procédé d'activation est mis en oeuvre en phase gazeuse.

Le catalyseur comprend généralement au moins un métal du groupe VIII et un support. L'élément du groupe VIII de la classification périodique des éléments est choisi parmi le fer, le cobalt et le ruthénium. De préférence, le métal du groupe VIII est le cobalt.
Le support du catalyseur utilisable dans le procédé d'activation selon l'invention comporte au moins un oxyde réfractaire généralement choisi parmi les oxydes de magnésium, d'aluminium, de silicium, de titane ou de zirconium pris seuls, en mélange entre eux ou avec des oxydes d'autres éléments de la classification périodique. Le support utilisé sera préférentiellement choisi dans le groupe constitué par : l'alumine, la silice, l'oxyde de titane, le charbon, les alumino-silicates, les argiles. Toutefois, tout autre composé pouvant servir de support peut également être utilisé. Le support peut être utilisé sous forme de poudre ou après mise en forme ; toute technique de mise en forme connue de l'homme du métier peut être envisagée.

De préférence, le catalyseur utilisé dans le procédé d'activation selon l'invention est préparé par imprégnation d'au moins un métal sur un support préformé. Une technique de préparation du catalyseur est l'imprégnation d'une solution contenant des particules d'oxyde de métal et/ou des particules de métal à déposer en suspension. Le solvant pourra être un solvant aqueux, par exemple l'eau, ou un solvant organique.

La teneur en métal du groupe VIII, exprimée en poids de métal par rapport au poids total de catalyseur est généralement comprise entre 0,1 et 50 %poids, préférentiellement entre 1 et 40 %poids, et de manière plus préférée entre 5 et 30 %poids.

Le catalyseur peut également contenir d'autres éléments additionnels tels que par exemple au moins un métal alcalin, des promoteurs tels que par exemple au moins un élément choisi dans le groupe constitué par le ruthénium, le cuivre, le molybdène, le tungstène, le tantale, le titane et le scandium.

La teneur en poids d'un élément additionnel par rapport au poids total de catalyseur est généralement comprise entre 0,01 et 10 %poids, de préférence entre 0,1 et 5 %poids. Ces éléments additionnels peuvent être introduits en même temps que le métal du groupe VIII ou dans au moins une étape ultérieure.

En résumé, le procédé selon l'invention est un procédé d'activation d'un catalyseur de synthèse Fischer-Tropsch comprenant au moins deux étapes :
- au moins une étape d'activation en présence d'hydrogène, ou d'un mélange d'hydrogène et de gaz inerte, et
- au moins une étape d'activation en présence de monoxyde de carbone ou d'un mélange de monoxyde de carbone et de gaz inerte.

Dans un premier mode préféré de réalisation, il peut s'agir d'un procédé d'activation d'un catalyseur de synthèse Fischer-Tropsch comprenant au moins deux étapes et dans lequel :
- la première étape (étape 1) est réalisée en présence d'hydrogène ou d'un mélange d'hydrogène et de gaz inerte.
- la deuxième étape (étape 2) est réalisée en présence de monoxyde de carbone ou d'un mélange de monoxyde de carbone et de gaz inerte.

Dans un deuxième mode préféré de réalisation, il peut s'agir d'un procédé d'activation d'un catalyseur de synthèse Fischer-Tropsch comprenant au moins deux étapes et dans lequel :
- la première étape (étape 1) est réalisée en présence de monoxyde de carbone ou d'un mélange de monoxyde de carbone et de gaz inerte.
- la deuxième étape (étape 2) est réalisée en présence d'hydrogène ou d'un mélange d'hydrogène et de gaz inerte.

De préférence, les étapes 1 ou 2 sont réalisées à une température comprise entre 10°C et 700°C, à une pression comprise entre 0,05 MPa et 30 MPa, à une vitesse volumétrique horaire comprise entre 20 et 100 000 h⁻¹.

Le procédé d'activation selon l'invention peut en outre comprendre une troisième étape d'activation en présence d'hydrogène ou d'un mélange d'hydrogène et de gaz inerte. Il peut aussi éventuellement comprendre en outre une troisième étape d'activation en présence de monoxyde de carbone ou d'un mélange de monoxyde de carbone et de gaz inerte.

D'une manière préférée, une purge par du gaz inerte est réalisée à l'issue de chaque étape du procédé selon l'invention.

Selon l'un des modes de réalisation du procédé selon l'invention, une partie voire la totalité des étapes peuvent être réalisées en phase gazeuse. Selon un autre mode de réalisation du procédé selon l'invention, une partie voire la totalité des étapes peuvent être réalisées en phase liquide. Dans ce dernier cas, la phase liquide est de préférence une coupe paraffinique. De manière plus préférée, ladite phase liquide comprend des hydrocarbures ayant au moins 5 atomes de carbone par molécule et manière plus préférée au moins 10 atomes de carbone par molécule.

Le procédé selon l'invention permet d'activer tout catalyseur de synthèse Fischer-Tropsch. Il est par exemple particulièrement bien adapté pour activer un catalyseur comprenant au moins un métal du groupe VIII et un support.

Les exemples qui suivent illustrent l'invention.

### Exemple 1 (selon l'invention) : catalyseur A

Un catalyseur de formulation Co/AI2O3 est préparé par imprégnation d'une solution de nitrate de cobalt sur une poudre d'alumine présentant une surface spécifique de 180 m²/g. Après imprégnation, le support est séché à 120°C puis calciné à 400°C.
La teneur en cobalt du catalyseur calciné est de 12,5 %poids.

Après calcination, 20 cm³ de catalyseur sont réduits sous hydrogène pur avec un débit de 20 litres/heure (20 I/h) et à une température de 500°C, à pression atmosphérique et pendant 5 heures. On réalise alors une purge sous azote (201/h) pour éliminer la totalité de l'hydrogène et la température est diminuée de manière concomitante jusqu'à 230°C.

Lorsque la température atteint 230°C, le catalyseur est placé sous flux de monoxyde de carbone pur (10 I/h), toujours à pression atmosphérique et pendant 5 heures. Une purge à l'azote est à nouveau réalisée (débit 20l/h), puis une circulation d'hydrogène (20 l/h), à pression atmosphérique et pendant 1 heure.

### Exemple 2 (comparatif) : catalyseur B

Le même solide calciné que dans l'exemple 1 (20 cm³) est réduit sous hydrogène pur (débit 20 I/h) à une température de 500°C, à pression atmosphérique et pendant 5 heures.

### Exemple 3 (comparatif) : catalyseur C

Un solide C est préparé selon la même méthode que dans l'exemple 1.
Après calcination, le catalyseur (20 cm³) est réduit sous un mélange d'hydrogène et d'azote (20 % volume d'hydrogène - 80 % volume d'azote, débit 40 I/h) à une température de 400°C, à pression atmosphérique et pendant 10 heures.

### Exemple 4 (selon l'invention) : catalyseur D

Un catalyseur de formulation CoRu/SiO₂ est préparé à partir d'un support de silice. Le cobalt est imprégné dans une première étape à partir d'une solution de nitrate de cobalt. Le solide est ensuite séché à 120°C et calciné à 400°C.
Le ruthénium est ensuite imprégné en solution aqueuse. Le solide est séché à 110°C et calciné à 300°C. La teneur en cobalt est de 15 %poids et la teneur en ruthénium est de 0,25 %poids.

20 cm³ de catalyseur sont ensuite réduits sous hydrogène pur (débit 20 I/h) à une température de 400°C pendant 5 heures. On réalise ensuite une purge sous azote (débit 20 I/h) pour éliminer la totalité de l'hydrogène et la température est diminuée de manière concomitante jusqu'à 220°C.

Lorsque la température atteint 220°C, le catalyseur est placé sous flux de monoxyde de carbone pur (10 I/h), à pression atmosphérique et pendant 5 heures. Une purge à l'azote (débit 20 I/h) est ensuite à nouveau réalisée.

### Exemple 5 ( comparatif) : catalyseur E

Le catalyseur E est préparé au moyen de la méthode de préparation décrite à l'exemple 4. Après l'étape de calcination à 300°C consécutive au dépôt de ruthénium, un traitement d'activation est entrepris sur 20 cm³ de catalyseur E. Ce traitement comprend une seule étape de réduction sous hydrogène pur (débit 20l/h) à une température de 400°C, à pression atmosphérique et pendant 5 heures.

### Exemple 6 (comparatif) : catalyseur F

Un catalyseur F (30 cm³) est préparé à partir du même support à base de silice que dans l'exemple 4. Dans une première étape, le cobalt est imprégné à partir d'une solution de nitrate de cobalt. Le solide est ensuite séché à 120°C, calciné à 400°C, puis réduit dans un réacteur tubulaire sous hydrogène pur (30 I/h) à 400°C et passivé sous oxygène.
Le ruthénium est ensuite imprégné en solution aqueuse. Le solide est séché à 110°C, calciné à 300°C et à nouveau réduit dans un réacteur tubulaire sous hydrogène pur (30 I/h) à 400°C et passivé sous oxygène. La teneur en cobalt est de 15 %poids et la teneur en ruthénium est de 0,25 %poids.

20 cm³ de catalyseur F ainsi préparés sont réduits sous hydrogène pur au débit de 201/h, à une température de 300°C et à pression atmosphérique pendant 5 heures.

### Exemple 7 : Tests catalytiques

Les catalyseurs A, B, C, D E et F dont les préparations sont décrites dans les exemples 1 à 5 ci-dessus sont testés en lit fixe phase gazeuse dans une unité fonctionnant en continu et opérant sur 20 cm³ de catalyseur après l'étape de réduction décrite dans chacun des exemples correspondant de 1 à 6.

Après élimination de l'atmosphère gazeuse au moyen d'une purge sous azote, les catalyseurs sont amenés aux conditions de test de la synthèse Fischer-Tropsch. Ces conditions de test sont les suivantes:
- Température : 210 ou 220°C,
- Pression : 2 MPa
- vitesse volumique horaire (VVH) : 1500 h⁻¹ (volume de gaz par volume de catalyseur et par heure)
- rapport molaire H₂:CO = 2 :1

**TABLEAU : Conversion du gaz de synthèse en hydrocarbures**

| **Catalyseur** | **Temp. (°C)** | **Conversion CO (% volume)** | **Distribution des produits formés (% poids)** | |
|---|---|---|---|---|
| | | | **C₁** | **C₅+** |
| A (invention) | 210 | 56 | **10** | **75** |
| B (comparatif) | 210 | 35 | **20** | **55** |
| C (comparatif) | 210 | 50 | **15** | **60** |
| D (invention) | 220 | 75 | **12** | **65** |
| E (comparatif) | 220 | 66 | **18** | **52** |
| F (comparatif) | 220 | 65 | **25** | **49** |

Les résultats du tableau montrent que le procédé d'activation selon l'invention conduit à un rendement en produits lourds amélioré par rapport aux procédés d'activation de l'art antérieur.

## Revendications

1. Procédé d'activation d'un catalyseur de synthèse Fischer-Tropsch comprenant au moins deux étapes :
- au moins une étape d'activation en présence d'hydrogène, ou d'un mélange d'hydrogène et de gaz inerte, et
- au moins une étape d'activation en présence de monoxyde de carbone ou d'un mélange de monoxyde de carbone et de gaz inerte,
dans lequel le catalyseur comprend au moins un métal du groupe VIII et un support, et ledit catalyseur n'est pas mis en contact simultanément avec l'hydrogène et le monoxyde de carbone.

2. Procédé d'activation selon la revendication 1 comprenant au moins deux étapes et dans lequel :
- la première étape est réalisée en présence d'hydrogène ou d'un mélange d'hydrogène et de gaz inerte.
- la deuxième étape est réalisée en présence de monoxyde de carbone ou d'un mélange de monoxyde de carbone et de gaz inerte.

3. Procédé d'activation selon la revendication 1 comprenant au moins deux étapes et dans lequel :
- la première étape est réalisée en présence de monoxyde de carbone ou d'un mélange de monoxyde de carbone et de gaz inerte.
- la deuxième étape est réalisée en présence d'hydrogène ou d'un mélange d'hydrogène et de gaz inerte.

4. Procédé selon l'une des revendications 2 ou 3 dans lequel les étapes sont réalisées à une température comprise entre 10°C et 700°C, à une pression comprise entre 0,05 MPa et 30 MPa, à une vitesse volumétrique horaire comprise entre 20 et 100 000 h⁻¹.

5. Procédé selon l'une des revendications 1 ou 2 comprenant en outre une troisième étape d'activation en présence d'hydrogène ou d'un mélange d'hydrogène et de gaz inerte.

6. Procédé selon l'une des revendications 1 ou 3 comprenant en outre une troisième étape d'activation en présence de monoxyde de carbone ou d'un mélange de monoxyde de carbone et de gaz inerte.

7. Procédé selon l'une des revendications 1 à 6 dans lequel une purge par du gaz inerte est réalisée à l'issue de chaque étape.

8. Procédé selon l'une des revendications 1 à 7 dans lequel la totalité des étapes est réalisée en phase gazeuse

9. Procédé selon l'une des revendications 1 à 7 dans lequel la totalité des étapes est réalisée en phase liquide.

10. Procédé selon la revendication 9 dans lequel la phase liquide est une coupe paraffinique comprenant des hydrocarbures ayant au moins 5 atomes de carbone par molécule.

## Patentansprüche

1. Verfahren zur Aktivierung eines Katalysators zur Fischer-Tropsch-Synthese, das mindestens zwei Schritte umfasst:
- mindestens einen Schritt zur Aktivierung in Gegenwart von Wasserstoff oder einem Gemisch aus Wasserstoff und Inertgas und
- mindestens einen Schritt zur Aktivierung in Gegenwart von Kohlenmonoxid oder einem Gemisch aus Kohlenmonoxid und Inertgas,
wobei der Katalysator mindestens ein Metall der Gruppe VIII und einen Träger umfasst und der Katalysator nicht gleichzeitig mit dem Wasserstoff und dem Kohlenmonoxid in Kontakt gebracht wird.

2. Verfahren zur Aktivierung nach Anspruch 1, das mindestens zwei Schritte umfasst, wobei:
- der erste Schritt in Gegenwart von Wasserstoff oder einem Gemisch aus Wasserstoff und Inertgas durchgeführt wird,
- der zweite Schritt in Gegenwart von Kohlenmonoxid oder einem Gemisch aus Kohlenmonoxid und Inertgas durchgeführt wird.

3. Verfahren zur Aktivierung nach Anspruch 1, das mindestens zwei Schritte umfasst, wobei:
- der erste Schritt in Gegenwart von Kohlenmonoxid oder einem Gemisch aus Kohlenmonoxid und Inertgas durchgeführt wird,
- der zweite Schritt in Gegenwart von Wasserstoff oder einem Gemisch aus Wasserstoff und Inertgas durchgeführt wird.

4. Verfahren nach einem der Ansprüche 2 oder 3, wobei die Schritte bei einer Temperatur im Bereich zwischen 10 °C und 700 °C, bei einem Druck im Bereich zwischen 0,05 MPa und 30 MPa, bei einer Raumgeschwindigkeit pro Stunde im Bereich zwischen 20 und 100.000 h⁻¹ durchgeführt werden.

5. Verfahren nach einem der Ansprüche 1 oder 2, das ferner einen dritten Schritt zur Aktivierung in Gegenwart von Wasserstoff oder einem Gemisch aus Wasserstoff und aus Inertgas umfasst.

6. Verfahren nach einem der Ansprüche 1 oder 3, das ferner einen dritten Schritt zur Aktivierung in Gegenwart von Kohlenmonoxid oder einem Gemisch aus Kohlenmonoxid und aus Inertgas umfasst.

7. Verfahren nach einem der Ansprüche 1 bis 6, wobei am Ende jedes Schritts eine Spülung mit Inertgas durchgeführt wird.

8. Verfahren nach einem der Ansprüche 1 bis 7, wobei die Gesamtheit der Schritte in gasförmiger Phase durchgeführt wird.

9. Verfahren nach einem der Ansprüche 1 bis 7, wobei die Gesamtheit der Schritte in flüssiger Phase durchgeführt wird.

10. Verfahren nach Anspruch 9, wobei die flüssige Phase ein Paraffinschnitt ist, der Kohlenwasserstoffe mit mindestens 5 Kohlenstoffatomen je Molekül umfasst.

## Claims

1. Process for activating a Fischer-Tropsch synthesis catalyst that comprises at least two stages:
-- at least one stage for activation in the presence of hydrogen, or a mixture of hydrogen and a cover gas, and
-- at least one stage for activation in the presence of carbon monoxide or a mixture of carbon monoxide and a cover gas,
in which the catalyst comprises at least one metal of group VIII and a substrate, and said catalyst is not brought into contact simultaneously with hydrogen and carbon monoxide.

2. Process according to claim 1 comprising at least two stages and in which:
-- the first stage is carried out in the presence of hydrogen or a mixture of hydrogen and a cover gas,
-- the second stage is carried out in the presence of carbon monoxide or a mixture of carbon monoxide and a cover gas.

3. Process according to claim 1 comprising at least two stages and in which:
-- the first stage is carried out in the presence of carbon monoxide or a mixture of carbon monoxide and inert gas,
-- the second stage is carried out in the presence of hydrogen or a mixture of hydrogen and inert gas.

4. Process according to one of claims 2 or 3, in which the stages are carried out at a temperature of between 10°C and 700°C, at a pressure of between 0.05 MPa and 30 MPa, at an hourly volumetric flow rate of between 20 and 100,000 h⁻¹.

5. Process according to one of claims 1 or 2 that also comprises a third activation stage in the presence of hydrogen or a mixture of hydrogen and a cover gas.

6. Process according to one of claims 1 or 3 that also comprises a third activation stage in the presence of carbon monoxide or a mixture of carbon monoxide and a cover gas.

7. Process according to one of claims 1 to 6, in which purging by a cover gas is carried out at the end of each stage.

8. Process according to one of claims 1 to 7, in which all of the stages are carried out in a gaseous phase.

9. Process according to one of claims 1 to 7, in which all of the stages are carried out in liquid phase.

10. Process according to claim 9, in which the liquid phase is a paraffinic fraction that comprises hydrocarbons that have at least 5 carbon atoms per molecule.
